# EUROPEAN PATENT SPECIFICATION

(11) **EP 4 175 681 B1**
(45) Date of publication and mention of the grant of the patent: **17.09.2025**
(21) Application number: 21739766.0
(22) Date of filing: 29.06.2021
(51) Int. Cl.: A61L 9/03, A61L 2/20, A61L 2/00, A23B 7/153, A23B 7/158

(54) **APPARATUS FOR VAPOURISING A LIQUID FOR SUPPLY TO AN ENVIRONMENT**
VORRICHTUNG ZUM VERDAMPFEN EINER FLÜSSIGKEIT ZUR VERSORGUNG EINER UMGEBUNG
APPAREIL POUR VAPORISER UN LIQUIDE À FOURNIR À UN ENVIRONNEMENT

(30) Priority: 01.07.2020 GB 202010067
(43) Date of publication of application: 10.05.2023
(73) Proprietor: Greenbean Scientific Limited, Rochester, Kent MEI 1UX (GB)
(72) Inventor: TAYLOR, Sarah, Sittingbourne Kent ME9 8HL (GB); MAY, James, Warwick Warwickshire CV34 4AB (GB); HARRISON, Robert, Warwick Warwickshire CV34 4AB (GB)
(74) Representative: Hancox, Jonathan Christopher
(86) International application number: PCT/GB2021/051646
(87) International publication number: WO 2022/003342

(56) References cited:
- WO-A1-2006/101055
- WO-A1-2020/119926
- US-A1- 2012 214 110
- US-A1- 2015 130 088

## Description

### Technical Field

The invention relates to apparatus for vapourising a liquid for supply to an environment. In preferred embodiments, the invention relates to apparatus for vapourising a liquid for supply to an environment for treating fruit or vegetables in low temperature and/or high air flow conditions.

### Background

Pre- and post-harvest strategies aimed at controlling diseases of food crops typically include the use of pesticides, specifically fungicides for controlling fungal pathogens.

Despite new advances in the development of environmentally- and food-safe fungicides, the control of commercially important plant pathogens is often not complete resulting in significant losses and wastage. This situation has been further exacerbated in recent years by the legislative removal of a number of fungicides (pre-and post-harvest). It is therefore essential that available fungicides are delivered in an effective and resource-efficient manner suitable for as wide a range of food crops as possible.

Producers continue to be reliant on a small number of postharvest chemicals that are typically applied as a spray, a drench or a dip during packhouse operations. In addition to using high volumes of water, these delivery systems are only suitable for a limited range of crops (e.g. citrus; pome fruit) that are tolerant to being submerged or sprayed with water postharvest. There is also an increased risk of cross-contamination during treatment and the build-up of resistance in pathogen populations. Disposal of the pesticide treated water after use is also a concern.

The disadvantages associated with the above delivery systems can be addressed through the application of antimicrobials as a vapour. Although a number of the existing technologies for creating a vapour have their merits, they also have limitations. In the case of thermofogging, the relatively high fogger exhaust temperatures can result in pesticide heat decomposition. This method of application may lead to non-uniform distribution of the active that can result in either too low a dose thereby compromising efficacy or too high a dose resulting in exceeding regulatory maximum residue limits.

International patent publication no. WO2013/128213 (AG Thames Holdings Ltd.) describes a dispersal unit and method for the treatment of perishable goods at different stages of the supply chain based on the controlled vapourisation and dispersal of a volatile antimicrobial "active material" within the storage volume. This method releases an antimicrobial active from a formulation loaded onto a solid support through gentle warming of the mixture. Whilst it addresses the issue of applying high temperatures to volatilise the active, it has the disadvantage that a significant proportion of the active remains bound to the support, making it inefficient, wasteful and costly. In addition, this method presents a risk of exposure of operators to antimicrobial materials whilst handling the cartridges.

A number of volatile material dispensers developed for a variety of purposes are known in the prior art. Such inventions may be air/room fresheners, fragrance dispensers, insect repellent devices, insect killing devices, electronic cigarettes, virtual reality experience devices. These volatile material dispensers are generally not suitable for working in refrigerated conditions such as those experienced in the perishable fruit and vegetable supply chain, for example, in precoolers, blast chillers, fruit stores, marine and truck containers/trailers etc. Problems which may occur in these conditions include: an insufficient rate of vapourisation, re-condensation of the volatile liquid back onto the dispenser and suboptimal dispersal of the volatile material in the treated environment.

The present disclosure describes a device for the vapourisation and delivery of liquid formulations into environments that addresses the disadvantages of the prior art. The device described herein provides beneficial delivery options for the controlled vapourisation and dispersal of antimicrobial materials (including pesticides and natural plant chemicals) and other chemicals (such as 1-methylcyclopropene, diphenylamine).

While a preferred use of the invention is for vapourising a liquid for supply to an environment for treating fruit and vegetables in chilled and/or high air flow conditions, the invention is not limited to such use. The invention will have wide application in many fields where the vapourisation of a liquid into an environment is required, and limitation of the invention to a specific application is not implied.

US 2015/0130088 (Joshi et al.) discloses a volatile substance delivery system including a container and a drop delivery system to deliver a drop of the volatile substance to a volatile substance delivery structure. WO 2020/119926 (CTR, LDA) discloses a device for evaporating volatile substances comprising a reservoir, a wick and a heating device. WO 2006/101055 (Kobayashi Pharma) discloses a liquid vaporizer having a container for storing the liquid to be vaporized and a container heater. US 2012/0214110 (Cagle et al.) discloses a container for a candle having a chimney-like assembly which separates inlet air from the active scent and accelerates active scent flow.

### Summary of the Invention

The present invention provides an apparatus for vapourising a liquid for supply to an environment in accordance with claim 1. Optional and preferred features are set out in the dependent claims.

Pre-heating of the inlet air, thereby delivering warm air to the wick, is a very advantageous feature of this aspect of the invention. Pre-heated inlet air can be used to control the rate of vapourisation in order to deliver the required dose or headspace concentration of the active material in the vapourised liquid. The warmed air acts as a carrier for the volatilised material. The warmed air helps to control heat loss at the wick. If the inlet air was not heated, it could draw heat away at the wick surface which would reduce the temperature of the liquid on the surface of the wick, potentially below the vapourisation temperature, which would reduce the rate of vapourisation or even stop vapourisation altogether. The warmed air may also warm the wick, which may in certain conditions mean that the wick heaters discussed below are not required.

As a further advantage, warmed inlet air helps to decrease condensation of the volatile material back onto the apparatus or onto surrounding surfaces once it has left the outlet, because the temperature of the outlet plume is higher and more consistent.

This aspect of the invention is useful in all environments, but is particularly useful in any environment where the temperature of the environment is below the phase transition temperature (liquid to vapour) of the liquid formulation, e.g. in chilled or refrigerated conditions.

The environment may be any space which is to be treated with the active material in the vapourised liquid and could be open, closed or semi-contained. A closed environment could be any closed space from a package (e.g. pallet hood) through to a room such as a warehouse, cold-storage facility, cooler, ripening room, store, shipment container, closed vehicle trailer, glass house, grain silo, or operating theatre. An example of an open environment is an outside agricultural setting, e.g. fogging grape vineyards, soil fumigation treatments etc. Another example would be in the creation of an aroma e.g. in retail or leisure space, outdoor cinema, etc.

The inlet air may be drawn in from the area surrounding the apparatus, i.e. from the same open, closed or semi-contained environment to which the inlet air and vapourised liquid mixture is supplied. However, the inlet air could alternatively be drawn in from a different location, such as externally to the closed environment if appropriate ducting is provided. Preferably however, the inlet receives inlet air from the environment to which the apparatus supplies the inlet air and vapourised liquid mixture. This has the advantage that the apparatus is self-contained and transportable.

The term "air" is to be construed broadly to include normal, unmodified atmospheric air (e.g. if the inlet air is drawn externally from the environment being treated) as well as treated or modified atmospheric air (e.g. if the inlet air is from the environment being treated). The gaseous composition may be the same as or vary from the composition of atmospheric air, and may for example contain a higher or lower percentage of certain gases, e.g. carbon dioxide or oxygen.

The feature of heating or warming the inlet air is to be construed broadly and means that the apparatus raises the temperature of the inlet air before it reaches the wick. The temperature rise may be only a relatively small amount (e.g. a few degrees C, such as about 2 degrees C, or up to about 10 degrees C) up to a relatively large temperature change (e.g. 10 degrees C or higher, or up to about 100 degrees C, or higher). The actual temperature rise will depend on the specific conditions in each case, including the vapourisation temperature of the liquid being employed, the inlet air temperature and the desired temperature of the outlet air/vapourised liquid mixture.

As mentioned above, the invention is particularly useful in chilled or refrigerated environments. Such environments may have a relatively low temperature, for example in the range of -1 to 15 degrees C.

The invention is also useful in environments where the temperature range changes, such as during precooling of fruit and vegetables (used to remove field heat) where the produce will be placed in a room e.g. forced air chiller, at ambient temperature and the produce item is reduced to typically 0 to 15 degrees C depending on whether the product is chill-sensitive. Similarly the device could be used in a ripening room, where the fruit is treated at e.g. 18 to 22 degrees C for a number of days. The produce is placed in the room at its recommended storage temperature (e.g. 0 to 15 degrees C) and then warmed at the higher temperatures for a number of days before being cooled in a blast chiller back to the recommended storage temperature (0 to 15 degrees C).

The duct path includes an inlet duct section between the inlet and the wick and the apparatus further includes an inlet heater in the inlet duct section for heating or warming the inlet air. The apparatus may further include an inlet duct section temperature sensor to provide temperature information to a control means for controlling the inlet heater. Preferably the heater is an electric heater.

The apparatus further includes a fan in the inlet duct section for drawing in the inlet air. A fan may provide an increased level of control over the airflow through the apparatus and past the wick. A fan may permit more inlet air to be drawn in, which increases the internal pressure in the apparatus and which will also increase the exit velocity of the plume and therefore the height of plume from the outlet. This may be particularly advantageous in treating high air flow environments.

In preferred embodiments, changing the rate of air flow through the apparatus will affect the rate of vapourisation of the liquid at the wick surface. There is a balance between the heated air temperature moving through the ducting, the temperature of the solution as it moves up the wick and the need for any localised heating of the wick (discussed below).

Although not to exclude other sources of inlet air, a preferred feature of the apparatus when the inlet air is drawn in from the area surrounding the apparatus is that the apparatus further includes an inlet plenum chamber external to or upstream of the inlet. This may take the form of a concave or recessed chamber for example. Preferably the inlet is located within the plenum chamber, recessed within it. Preferably, the plenum chamber is formed on the underside of housing. The plenum chamber may be formed in the housing or in a separate structure such as a base attached to the housing. In a preferred embodiment, the housing or base has a plurality of feet to space the apparatus from the floor or surface on which it stands and the plenum chamber is within the area defined by the feet, preferably on the underside of the housing or base, facing the surface. This arrangement forms an air gap between the floor/surface and the inlet and allows air from any direction to flow freely between the feet into the plenum chamber. The plenum chamber reduces the local velocity of the air, to create a localised, slower-moving volume of air from which the inlet air is drawn into the apparatus.

This preferred aspect of the invention is particularly useful when treating high air flow environments. The plenum chamber forms a localised pocket, positioned out of the line of the air flow, which means that the local air speed in the pocket moves more slowly making it easier for air to be drawn in, e.g. by an inlet fan (if present). Another advantage of the plenum chamber when a fan is employed is that the fan is not forced by the environmental air flow in certain orientations. Therefore, the rotational alignment of the apparatus is not critical to achieving a consistent inlet air flow and therefore a consistent performance.

The duct path includes an outlet duct section between the wick and the outlet and the outlet duct section is configured to accelerate the flow of the mixture of inlet air and vapourised liquid away from the wick towards the outlet.

The configuration of the outlet duct, at least in preferred embodiments, increases the velocity of the mixed vapour flow out of the device, so that the vapour plume extends further from apparatus before dispersing. This helps to prevent the plume losing its energy which could potentially cause recondensation of the liquid onto the external surface of the apparatus or on surfaces in the immediate vicinity of the device, which would result in poor performance, e.g. in low temperature environments. This aspect of the invention is useful where there is a large temperature difference and the ambient air flow has capability to draw the energy from the vapour plume. Projecting the vapour into the headspace encourages more widespread dispersing.

This aspect of the invention is also advantageous in high air-flow environments. Although the use of an outlet fan in the outlet duct section between the wick and the outlet is not excluded in the present invention, the configuration of the outlet duct to increase the velocity of the mixed vapour flow out of the device means that an outlet fan can be avoided.

The outlet duct section preferably includes a conical chamber which reduces in cross-sectional area in the direction of flow towards the outlet to accelerate the flow of the mixture of inlet air and vapourised liquid away from the wick towards the outlet. In a particularly preferred embodiment, the chamber is a truncated cone. In addition to the advantages of the function of the outlet duct section discussed above, a small chamber outlet orifice also reduces the amount of heat lost out of the top of the device. Keeping the apparatus warm is important to maintaining performance.

Preferably the outlet duct section includes a straight section downstream of the conical chamber. The straight section is preferably vertical and acts as a chimney after (and above) the conical chamber. The outlet is orientated vertically in order to project the vapour into the headspace of the environment and encourage mixing in the environment air. The chimney (which preferably protrudes out of the apparatus) helps to further increase the flow rate through the system and achieve a higher dispersal height of the vapour plume, thereby moving the plume away from the device.

If it is desired to target a specific area of the environment, a longer chimney or a chimney extension could be used, e.g. up to about 1 metre in length, or longer depending on the specific application. The chimney extension could be useful to treat specific areas of the environment, for example directly onto or under a pallet, in to a pallet hood or fruit bin etc.

In a preferred embodiment, the inlet and outlet are arranged on the same vertical axis with the outlet above the inlet. The conical chamber, chimney and/or the chimney extension, if present, may also lie on the same vertical axis between the inlet and outlet, as may one or more of the other components discussed below. A general flow of air from inlet to outlet in a vertical, upward direction, with minimal changes in direction, provides an optimised, efficient system.

The apparatus preferably further comprises a wick heater for heating the wick at or adjacent to the exposed end of the wick. This feature assists with the capillary uplift of liquid to the exposed wick end and increases the evaporation rate. The apparatus may further include a wick or wick heater temperature sensor to provide temperature information to a control means for controlling the wick heater.

The apparatus may be provided with a plurality of wicks, e.g. 2 or more, or from 2 to 10 wicks. A preferred number of wicks is four. A single wick heater may be provided for a plurality of wicks, or individual heaters may be provided for each wick. Each wick may be independently temperature controlled with individual temperature sensors to regulate the conditions at each wick end, or all wicks could be globally controlled with a single temperature sensor.

The wick may be made from any suitable material to perform the required function of delivering the liquid from the reservoir to the point of evaporation. The choice of wick material is dependent on a number of factors, including the formulation and properties of the liquid, the wicking rate required, and the thermal or chemical resistance (the resistance of the wick material to the formulation or the heat applied, e.g. in the reservoir or by the wick heaters). Suitable materials include porous or sintered plastics, glass-sintered fibres, sintered carbon, bundled or woven natural fibres such as cotton and linen.

The density of the wick material may also be dependent on similar factors to the material choice. A preferred density range has been found to be from about 0.25 g/cm³ to about 0.5 g/cm³ although densities outside this range may be suitable. The wick is preferably self-supporting. In other words, the wick has sufficient inherent rigidity that it can extend in a vertical direction while being held at one point only. The wick is preferably cylindrical and a preferred diameter is about 8mm although other diameters will function equally well. With a cylindrical wick the wick heater preferably extends around the wick and has a circular aperture to receive the wick.

The wick heater is preferably mounted in an apertured wick heater plate. The one or more apertures in the wick heater plate allow the inlet air (which may be heated) to pass around the exposed wick end such that the inlet air mixes with the evaporating liquid at or downstream of the heater plate. The arrangement of one or more wick heaters in the heater mounting plate and their proximity to the exposed end of the wick is particularly advantageous in terms of capillary uplift of the formulation and the evaporation rate. The preferred arrangement is particularly advantageous in terms of uniformity of the distribution of heat around the circumference of the wick.

Apertures may be provided in the wick heater plate itself. Apertures may alternatively or in addition be provided between the wick and the heater, forming a relatively narrow air gap. When the wick is cylindrical and the heater has a circular aperture to receive the wick, the air gap will be annular.

The duct path preferably has a wick duct section between the inlet duct section and the outlet duct section, the exposed end of the wick extending into the wick duct section. The one or more apertures in the wick heater plate preferably form part of the wick duct section.

A wick mounting plate may be provided for mounting the wick. The wick mounting plate may be provided with a grip to hold and align the wick with the heater, in the correct position in the wick duct section. The grip may be referred to as an interference mechanism. The grip maintains the wick such that the wick is coaxial with the wick heater axis.

In a preferred embodiment, the wick mounting plate is spaced from the wick heater plate. The space between the plates preferably forms part of the wick duct section. The wick, wick mounting plate and wick heater plate may be considered to form a wick assembly.

The apparatus further includes a reservoir heater for heating the liquid to be vapourised. Such a heater can assist with capillary uplift of the liquid to the exposed wick end. This may be very advantageous in a low-temperature environment. As heat is transferred to the liquid formulation its viscosity decreases and the rate of transfer of the liquid from the proximal to the distal end of the wicks is increased. The apparatus may further include a reservoir temperature sensor to provide temperature information to a control means for controlling the reservoir heater.

A preferred combination of all three heaters mentioned above (inlet, wick, reservoir) has been found to be particularly advantageous. This combination allows improved control and optimisation of the vapourisation process, providing a fine level of adjustment which can take into account the ambient environment.

For increased ease of use, e.g. for simpler refilling or maintenance, the apparatus may include a removable cartridge assembly which includes the reservoir and the wick. Other components may be included in the cartridge assembly, including the wick heater, wick temperature sensor, wick heater plate, wick mounting plate, wick duct section, reservoir temperature sensor, outlet duct, conical chamber, chimney.

The housing is preferably cylindrical. The overall shape of the device has been derived to ensure there is sufficient insulation around the components to minimise heat loss to the surrounding environment.

In at least preferred embodiments, the rate of vapourisation is controlled by the temperature of the inlet air delivered to the top of the one or more wicks (controlled by the inlet heater), the air flow rate through the device (which may be controlled by an inlet fan), the temperature of the formulation in the reservoir (controlled by the reservoir heater) and the temperature of the wick heater(s). The rate of vapourisation is also controlled by the properties of the wick material and the composition of the formulation.

The apparatus may be suitable for use in the following situations:
- disinfecting a contained environment such as a storage area, transport container, precooler, ripening room, degreening room or glasshouse using an antimicrobial formulation administered as a vapour using the apparatus;
- treating plants or plant parts with an antimicrobial liquid formulation to inhibit plant pathogens of agricultural and horticultural crops using the device in a contained environment. The plants or plant parts may include fruit, vegetables and cut flowers and the contained environment may comprise a storage area, marine container, precooler, ripening room, degreening room, pallet hood or enclosed bin.

The present invention discloses a device/apparatus for vapourising liquid formulations. The device comprises a housing for enclosing/receiving a cartridge assembly comprising a refill/vessel containing a liquid formulation and wick(s). The present disclosure is directed to heater arrangements, air flow pathways, wick arrangements, vapourising devices and the shape of housing including the exit configuration. These disclosures facilitate the controlled vapourisation of liquid formulations, optimisation of the vapour plume, and reduces condensation of the volatile material back on to the device after that volatile material is emitted from the device. This is advantageous in chilled contained environments, such as cold stores and refrigerated transport containers.

The volatile material dispenser may be placed in a contained environment such as a marine container or refrigerated store. The device delivers a vapour, for example an antimicrobial vapour, that is used to disinfect a contained environment and/or inhibit the growth of plant pathogens of plants or plant parts e.g. fruit held in a contained environment. The device is particularly advantageous, but not limited to, the vapourisation of liquid formulations in low temperature environments, where the controlled vapourisation and dispersal of volatile materials is challenging.

The overall shape of the device has been derived to ensure that there is sufficient insulation around the cylindrical flask (cartridge), in order to reduce the loss of heat from the device to the external environment and in particular to ensure that the temperature of the formulation in the cartridge is maintained and the ambient temperatures around the wicks and wick heaters are high enough to ensure vapourisation of the formulation from the wick surface. This is particularly advantageous when the device is employed in a refrigerated environment. The housing contains insulation, such as a combination of thermal insulation boards (polyisocyanurate rigid foam insulation) and fibreglass insulation that surround the cartridge.

The device is cylindrical in shape and may have a footprint diameter of about 400 mm and overall height of about 370 mm. The dimensions of the device may be changed to fit a specific space or application. For example, the size of the device may be reduced or increased to fit a forced air cooler, marine container, operating theatre, glasshouse, cold store, tent, packaging unit, silo or fruit bin.

The device comprises a heater tank and lid that are constructed from stainless steel on the basis of its chemical and heat resistance and its suitability for use in a range of temperature environments.

The device has a foot arrangement designed to raise the base of the device off the surface of the floor, whilst maintaining stability. The design of the base of the device is integral to its functionality. The foot may be replaced with an alternative construction, so long as functionality is maintained. For example, wheels may be provided to increase the mobility of the device.

The apparatus may be affixed to a wall, ceiling or fittings e.g. chiller units, grills, albeit there may be a need to add features to the device to reduce the local airspeed at the outlet nozzle, for example by the inclusion of baffling around the device. Lower local windspeed enables the vapour plume to efficiently transfer into the headspace of the convection currents, or forced air currents, inside chillers. This promotes a wider spread through the headspace.

The device is arranged to hold a single cartridge assembly. The apparatus may be arranged to hold multiple cartridges or different volume cartridges for larger or smaller treatment spaces, or multiple devices may be used.

The nozzle outlet at top of the device is orientated vertically in order to project the vapour into the headspace of the container and encourage mixing. The nozzle outlet may be replaced with alternative fittings, such that the vapour may be directed through a pipe or duct and released into a smaller contained environment, such as a pallet hood, shroud, enclosed fruit bin or other container. There will be a need to make sure the wall temperatures of any ducting or piping are not too low to prevent pre-mature condensation of the formulation. This could be addressed by temperature controlled ducting.

The device is powered by an electrical supply source, which is controlled by a separate control unit connected to the device through a series of wired connections. The aim is to use rechargeable battery supply, so that the device can be positioned anywhere in a storage space without the problem of leads getting in the way. There is also the opportunity for the device to be controlled from outside the treatment container/space through a wireless WAN. In the absence of wireless connection to the internet, the unit may be controlled through a wired LAN.

The housing of the apparatus is configured to receive a cylindrical cartridge assembly comprising: a cylindrical cartridge that contains the liquid formulation; wicks and wick vapourising heater arrangement; and a vapour outlet fitting. The housing has a cup receptacle/crucible that locates and positions the cartridge into the housing. The cup receptacle/crucible is located in the central core of the heater tank and the body of the cartridge sits in the crucible.

The reservoir includes a heater arrangement mounted onto the crucible that warms the liquid formulation in the reservoir. Heaters are embodied on the device by using one or more silicone heater mats. These mats are adhesively bonded to the external surfaces of the crucible (mounted in the device). The crucible is preferably metallic so that it will conduct the heat from the heater mats through the crucible wall and into the cartridge flask. The gap between the crucible and the cartridge is small to encourage a good rate of heat transfer through conduction. This gap may be liquid-filled to increase thermal transfer.

The cartridge flask is fitted with a thermocouple that measures the temperature of the solution in the flask. This temperature provides an input into a closed loop setpoint controller that regulates the applied power to the heaters, controlling the formulation temperature. The heaters (heating mats) in the housing warm the liquid formulation in the body of the refill/cartridge. As heat is transferred to the liquid formulation its viscosity decreases and the rate of transfer of the liquid from the proximal to the distal end of the wicks is increased. The warming of the liquid in the body of the cartridge assists with capillary uplift of the liquid formulation to the wick heater vapourisers.

The temperature of the heaters in the housing may operate from 0 to 70 degrees C. Lower temperatures induce a slower rate of warming of the liquid, whereas higher temperatures induce faster warming of the formulation (heat transfer) and capillary flow up the wick. The temperature of the heaters can be used to control the rate of vapourisation of the liquid formulations. Although a specific heater type is disclosed herein, other heaters known in the art may be used, for example heater coils.

The cartridge assembly may comprise a cartridge with a volatile material therein, wicks (e.g. 4), vapourising heater and outlet configuration. The cartridge assembly is adapted for use with the vapourising device, whereby the body of the cartridge container (flask) is received and enclosed in an internal cavity (e.g. the crucible) of the heater tank of the housing.

Features of the cartridge assembly and housing allow the cartridge to be easily inserted into and removed from the housing without operator contact of the wick or formulation.

The cartridge/refill is preferably a cylindrical container such as a flask, with an outside diameter of about 70 mm and height of about 60 mm which is inserted into the central core of the device. The dimensions of the container/cartridge may be increased or decreased according to final application. The cartridge is constructed from stainless steel, but may be constructed from other materials (e.g. heat and chemically resistant plastic, glass). The cartridge contains a liquid formulation (e.g. up to 200ml) and four semi-porous wicks. The inclusion of four wicks in the present embodiment ensures there is sufficient capillary lift capacity.

In the present embodiment, the wicks are semi-porous. The choice of wick (material; wick dimensions; shape e.g. tubular/cog) is dependent on the intended use of the device (including the composition of the formulation and rate of wicking required). The semi-porous wicks are disposed within the body of the cartridge such that the proximal ends of the wicks are arranged to submerge in the formulation and the distal ends of the wicks protrude above the top of the cartridge (flask).

The top wall of the cartridge is a fitting that comprises the wick holders that support and align the wicks in the cartridge with the wick heaters. The wick holder fittings provide an interference fit to the outside diameter of the wick. This interference fit (retaining mechanism) is used to control the position of the wick in the cartridge. The distal end of the wicks protrude through the apertures of the wick holders and wick volatilisation heater devices that are set in the cartridge assembly. The cartridge assembly supports four wick vapourising heaters in a plate. The vapourising heaters are arranged to promote vapourisation of the liquid formulation at the distal end of the wicks (the proximal end is submerged in the liquid formulation).

The wick heaters are comprised of a length of high resistance heating wire, with each heater have a typical resistance of 30 kOhms, encapsulated into a heat resistant polymer. Each one of the four heaters are mounted into a heater mounting plate and provide radiant heating around a plurality of sides of the wicks. Although a specific wick vapouriser heater arrangement is disclosed herein, other heaters known in the art may be used, and or the number and arrangement of heater devices may be amended.

In a preferred embodiment the wick heater plate is mounted onto the top of the cartridge subassembly. There is an air gap between the top of wick holder support and the base of the heater plate. The heater plate arrangement is disposed near the distal end of the wick, and a radial air gap is formed between the heater arrangement and the wick. Heat from the vapourising heaters travels inwardly through the air gap toward the wick through conduction and radiation.

The current arrangement of the vapourising heaters in the heater mounting plate and their proximity to the distal end of the wicks is particularly advantageous in terms of uniformity in the distribution of heat around the circumference of the wick and the ability of the heaters to apply a greater or lesser amount of heat as required.

The wick heaters increase the temperature of the formulation on the surface of the wick by radiation and conduction, to a level close to its phase transition temperature. The surface temperature of the wick heaters is controlled by a closed loop set point controller using a thermocouple as its input, to regulate the applied power to the heaters controlling the wick surface temperature. Typically the temperature of the wick heaters may range from 0 to 70 degrees C. Lower temperatures induce slower evaporation/volatilisation of the formulation, whereas higher temperatures induce faster evaporation/volatilisation of the formulation.

It may be possible to adjust the height of the exposed wick protruding above the top of the cartridge flask and its engagement radially and axially into the heater.

Upon insertion of the cartridge assembly in the device, the top portion of the wick is disposed adjacent the vapour heater devices, such that the warmed formulation in the cartridge container moves through the wick and is volatilised by the wick heater devices.

The vapourised material exits the cartridge assembly via an outlet fitting. The outlet fitting sits above and encloses the wick heaters. The fitting comprises an inverted funnel-shaped chamber that directs the vapourised material through a chimney shaped nozzle extension at the top of the device.

The geometric constriction of the outlet accelerates the airflow though the outlet orifice. The particular design of the outlet fittings disclosed here ensure a high dispersal height of the plume that enables improved mixing of the vapourised material in the external environment. Furthermore, the geometry of the outlet fittings reduce the risk of the vapourised material sinking back onto the device due to cooling/condensing. Thus the present configuration is particularly advantageous in quickly and efficiently dispersing the volatilized material, thereby reducing condensation of that material on the dispenser and in the vicinity of the dispenser, particularly in a chilled environment.

The geometry of the nozzle may be changed to adjust the characteristics of the plume that is emitted from the device.

The cartridge flask and wicks may be formed as a separate or reusable sub assembly that is secured to the heater plate sub assembly. The cartridge may be refillable or single use.

As discussed above, one aspect of the device is the delivery of warmed air to the wicks surface to promote vapourisation of the liquid formulation. Warmed air is more efficient than cold ambient air at vapourising the formulation because warmed air draws less heat energy from the wicks to locally heat the air. It ensures that as air passes, the formulation is entrained into the airflow path. If the air was cold, the air flow would draw heat energy away from the wick surface, which in turn would reduce the temperature of the solution on the surface of the wick, potentially to a level below the vapourisation temperature. Warmed air delivery is particularly advantageous for vapourising formulations in chilled environments, such as cold stores, refrigerated containers.

The foot arrangement at the base of the device provides an air gap between the floor and the inlet (aperture/port). This plenum gap currently embodied by a concave chamber, allows ambient air from any direction to flow freely underneath the foot into the plenum chamber, this is advantageous in terms of reducing its local velocity.

Ambient air is drawn into the device by a fan through an inlet (aperture/port) at the top of the concave chamber (of the heater foot). The device inlet fan is speed controlled and allows the adjustment of the volumetric airflow of ambient air into the base of the device through the aperture (port/opening) in the base of the housing.

Ambient air is drawn into a heater duct at the base of the device and passes over a heater, such as a strip heater (200W), which increases the air temperature (e.g. from 2-3 degrees C typical fruit store temperature) to a user controlled set point (e.g. 70 degrees C). The warmed air temperature is controlled by thermocouples mounted in the duct, which are used as input to a closed loop temperature controller regulating the power applied to the heater to maintain the temperature set point. The thermocouples are mounted in the duct above the duct heater, adjacent to the wall of the crucible/cup that receives the cartridge.

The air ducting delivers warmed ambient air to the top of the heater tank. In order to promote vapourisation of the liquid at the wick surface, the warmed air is ducted to flow across the top surface of the cartridge. The warmed air then rises up around the wicks and passes through the air gap between the outer diameter of the wicks and the inner diameter of the wick heaters. Air that does not pass through these gaps due to the geometry constriction then flows externally to the wick heaters through gaps in the heater mounting plate.

The warm air with the entrained formulation then combines with the air that has passed through the heater plate, and exits through the outlet fittings.

The volatile liquid in the reservoir may be any type of volatile material adapted to be dispensed into an environment. For example, the volatile liquid may include or comprise any of the following:
- an antimicrobial, antibacterial, or antiviral agent
- an essential oil or component(s) of essential oil(s)
- a mineral or vegetable oil
- ethanol
- a fungicide, such as fludioxonil, imazalil, pyrimethanil or thiabendazole
- an insecticide, an insect repellent or an insect attractant
- an antioxidant; a sprout inhibitor; 1-methylcyclopropene (1-MCP); a ripening agent
- a disinfectant or sanitizer
- a pharmaceutical

The device is suitable for the controlled vapourisation of liquid formulations in open or contained environments and particularly, but not limited to, chilled or refrigerated environments or high air flow environments. Specific examples of applications include:
- A device for the controlled delivery of an antimicrobial material, such as essential oil(s) or component(s) of essential oil(s), in an enclosed space for the post-harvest treatment of fungal pathogens on fruit, vegetables, cut flowers, seeds, grains, cocoa and coffee. The contained environment, could include a fruit bin, pallet shroud, marine container, precooler, truck trailer, high care facility, ripening room, degreening room, storage room.
- A device for the controlled delivery of a fungicide such as fludioxonil, imazalil, pyrimethanil and thiabendazole in an enclosed space for the pre- and post harvest treatment of fungal pathogens on fruit, vegetables, cut flowers, seeds, grains, cocoa or coffee. The contained environment could be a glasshouse, silo or store.
- A device for the controlled delivery of volatile materials for use as an insecticide, insect repellent or insect attractant in a variety of environments.
- A device for the controlled delivery of volatile materials, such as antioxidants; sprout inhibitors; 1-methylcyclopropene (1-MCP); a ripening chemical, for the treatment of plants and plant material.
- A device for the controlled delivery of disinfectants or sanitizers for disinfecting a contained environment, such a marine container, truck trailer, high care facility, pre-cooler, ripening room, degreening room, storage facility, glasshouse, veterinary or medical environment (such as an operating theatre).
- A device for dispensing a fragrance or scent , e.g. in a museum, retail space, cinema.
- A device for dispensing a pharmaceutical.

### Brief Description of the Drawings

An embodiment of the invention will now be described by way of example only and with reference to the accompanying drawings, in which:
Fig. 1 shows a perspective exterior view of a vapourising device in accordance with the invention;
Fig. 2A shows a cross-sectional view through the device of Fig. 1;
Fig. 2B shows a cross-sectional view through the device of Fig. 1 at 90 degrees to Fig. 2A;
Fig. 3 shows a cartridge assembly for use in the device of Fig. 1;
Fig. 4 shows a cross-sectional view through the cartridge assembly of Fig. 3;
Fig. 5 shows a detailed view of the wicks and wick heater plate in the cartridge assembly;
Fig. 6 shows a cross-sectional view through the device indicating the airflow paths;
Fig. 7 shows detailed view of the airflow into the cartridge assembly and past the wicks and wick heater plate;
Fig. 8 shows a vapour plume profile without an outlet duct fitted; and
Fig. 9 shows a vapour plume profile with an outlet duct fitted.

### Detailed Description of a Preferred Embodiment

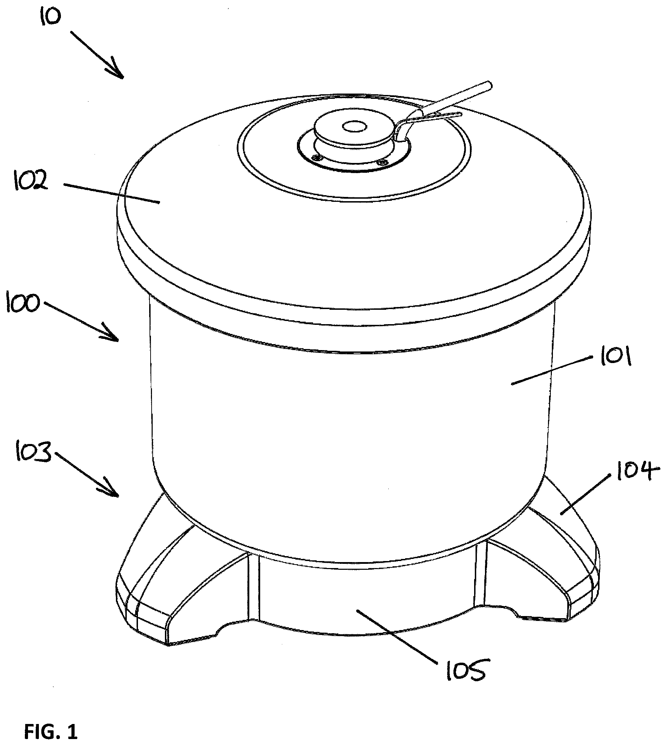

With reference to Fig. 1, an external view of a vapourising device 10 in accordance with the invention is shown. The device has a housing 100 comprising a cylindrical body 101 and a circular lid 102. Housing 100 is mounted on a base 103 which is generally circular and has three equally-spaced feet 104.

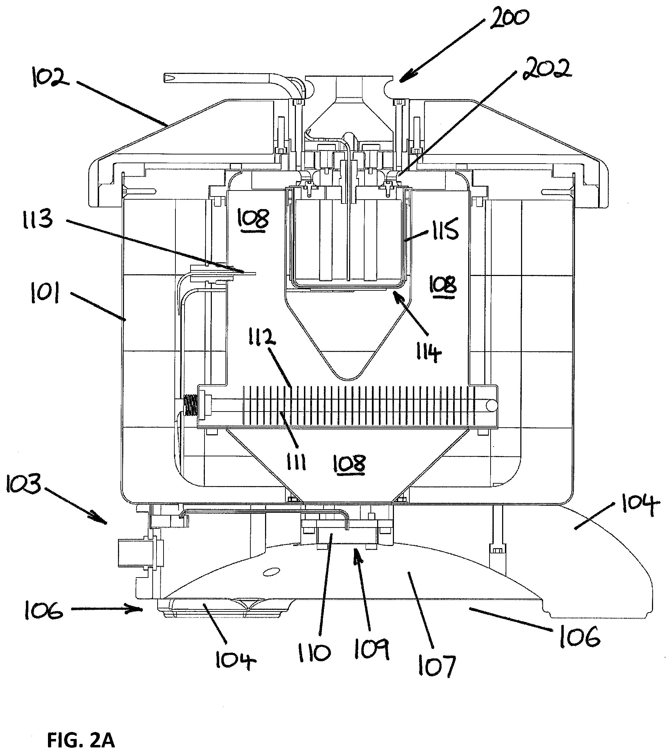

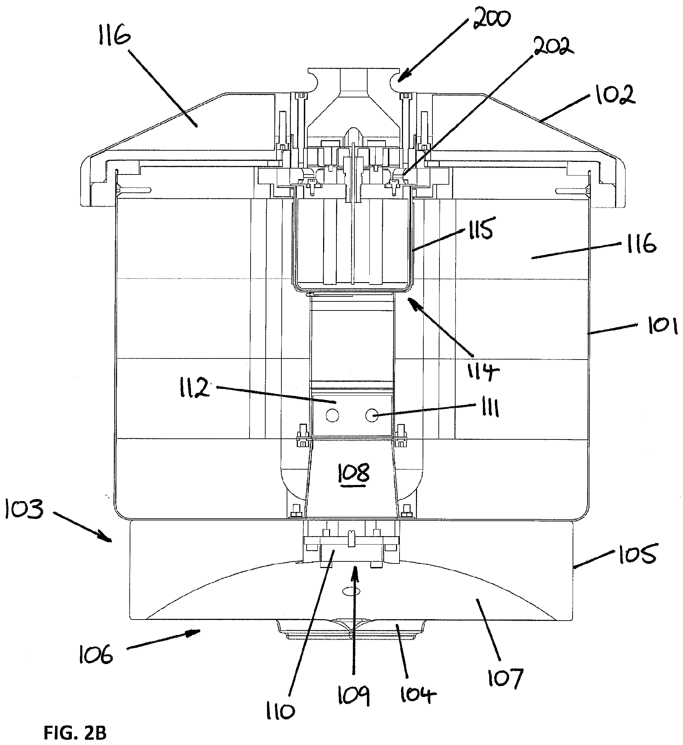

Turning to Figs. 2A and 2B, it can be seen that feet 104 project downwards further than the side wall 105 of the base so that three air gaps 106 are formed between the surface on which the device is standing and the side wall 105. Air gaps 106 lead to inlet plenum chamber 107 which is formed in the underside of the base, and is a generally concave, semi-spherical chamber. As discussed above, the plenum chamber reduces the local velocity of the air, to create a localised, slower-moving volume of air from which the inlet air is drawn into the device.

Plenum chamber 107 leads to inlet duct section 108 which is the first part of the duct path through the device and starts with inlet 109 and ends with the inlets 202 to the cartridge assembly 200 discussed below. An inlet fan 110 is provided at the inlet 109 to draw air in from the plenum chamber.

Inlet duct section 108 initially expands to slow the inlet air down for heating by means of electric inlet heater 111 which is provided with a plurality of fins 112. Inlet duct section temperature sensor 113 downstream of the heater 111 provides temperature information to a controller (not shown) for controlling the inlet heater.

Downstream of the heater 111, inlet duct section 108 splits into two paths and passes either side of a central holder 114 for the cartridge assembly 200, discussed further below. Holder 114 is also referred to as a container or crucible and is located in the central core of housing 100, bisecting the inlet duct section 108. This part is therefore very well insulated from the external environment. Holder 114 is provided with a reservoir heater 115 to heat the liquid being vapourised.

In order to minimise heat loss from the device, particularly if it is to be used in a low-temperature environment, the body and lid are fitted with insulation material 116 (e.g. foam board or mineral wool insulation).

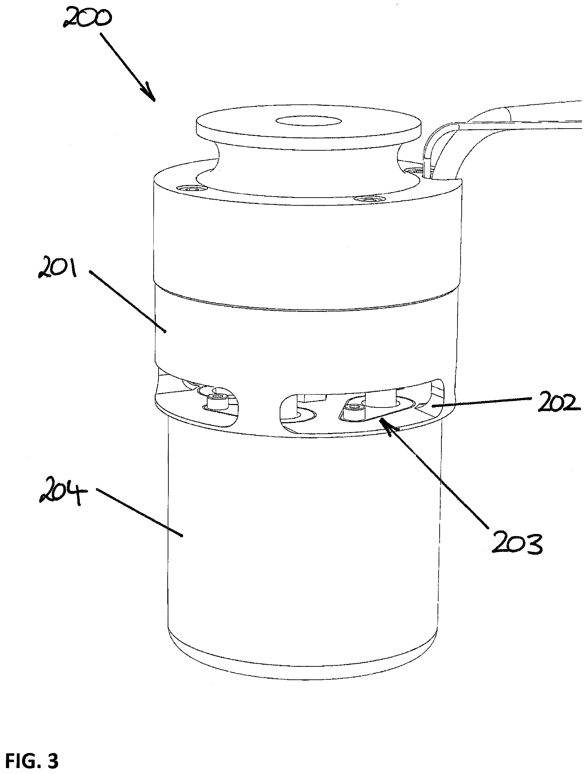

Cartridge assembly 200 is best described with reference to Figs. 3-5. Cartridge assembly 200 has a main body 201 in which are formed a plurality of inlets 202 which lead to wick duct section 203, which is the second part of the duct path through the device. The wick duct section is the section of the duct path which passes through the main body 201.

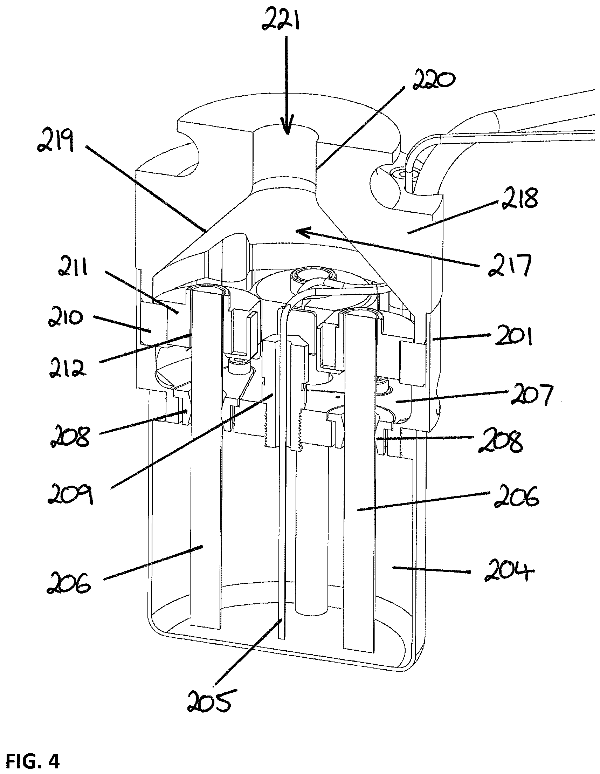

Cartridge assembly 200 has a reservoir 204 which contains the liquid to be vapourised in use. A reservoir temperature sensor 205 (Fig. 4) provides liquid temperature information to a controller (not shown) for controlling the reservoir heater. Cartridge main body 201 is removable from the reservoir 204 so that the reservoir can be refilled or maintenance of the components carried out.

Fig. 4 shows the internal components of the cartridge assembly 200. Four cylindrical wicks 206 are provided. The wicks are mounted with their major axes in a vertical orientation in a wick mounting plate 207 which is a generally circular plate covering the top of the reservoir 204. The wick mounting plate is provided with a gripping insert 208 for each wick. Each insert 208 has an aperture for the wick to pass through and a gripping surface on the side of the aperture (which may be a pinch-point, an interference fit, or an angled or ridged surface) which holds the wick and maintains the correct vertical alignment. The electrical connection for the reservoir temperature sensor 205 also passes through the wick mounting plate 207 through guide 209 mounted into the mounting plate.

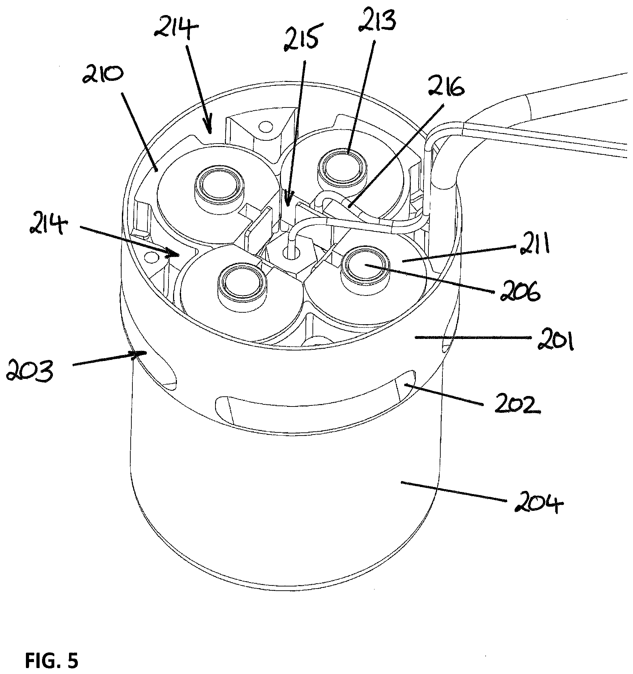

Spaced apart from the wick mounting plate, vertically above, is the wick heater plate 210. This can be seen in Figs. 4 and 5. Wick heater plate 210 comprises four wick heaters 211. Each wick heater is a generally cylindrical structure with a central, circular passage 212 to receive a wick. The passage diameter is configured to be slightly larger than the diameter of the wick so that an annular aperture 213 is formed allowing air to pass through the wick heater plate, between the heater and the wick. Other apertures are also provided in the wick heater plate, including peripheral apertures 214 and central aperture 215. All three apertures combine to allow inlet air to pass through the wick duct section 203, from inlets 202 through the wick heater plate 210, so that the inlet air passes the exposed wick ends and mixes with the vapourised liquid.

A wick heater temperature sensor 216 provides temperature information to a controller (not shown) for controlling the wick heaters 211.

With reference to Fig. 4, once the inlet air has passed the wicks and becomes a mixture of inlet air and vapourised liquid, the gas stream enters the outlet duct section 217 which is also part of the cartridge assembly 200 and is the third part of the duct path through the device. Outlet duct section 217 is formed in outlet body 218 which is removable from the cartridge main body 201. Outlet body 218 includes conical chamber 219, straight section 220 and outlet 221.

The combination of the conical chamber 219 (which is an upturned truncated cone) and the straight section 220 acts as a chimney and serves to accelerate the flow of the gas mixture away from the wicks and to the outlet 221, as discussed further above and below.

As an example of the preferred geometry for the conical chamber 219, the cone may taper from a diameter of about 70mm to about 17mm, with an angle of approximately 45 degrees. The height of the cone is about 30mm. The length of the chimney is about 15mm. Different dimensions are of course possible depending on the scale of the device, which will still be within the scope of the invention.

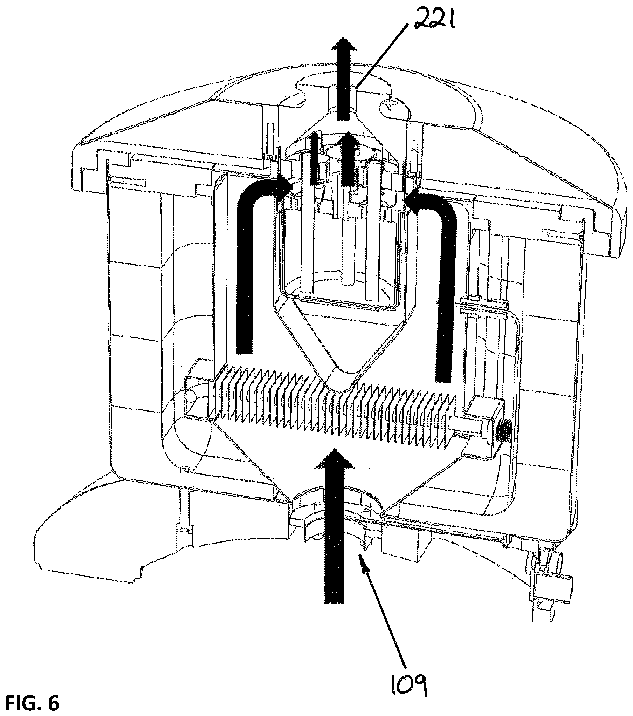

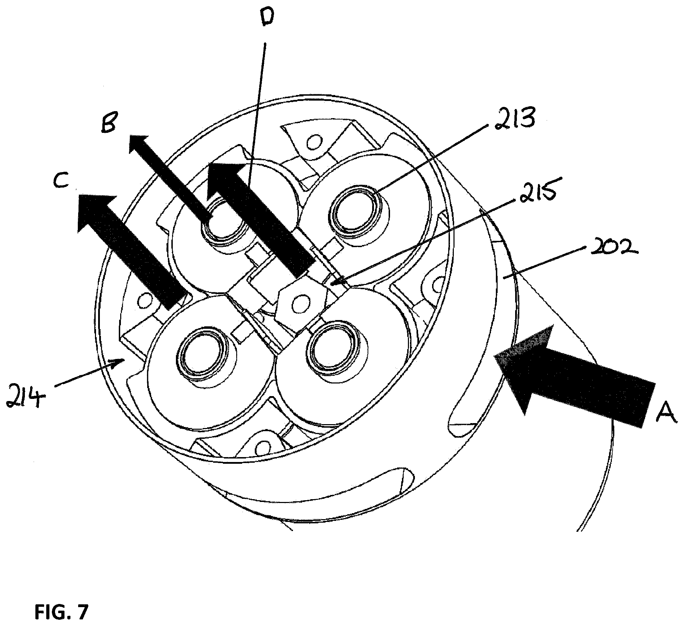

The flow of inlet air and inlet air/vapourised liquid mixture through the duct path of the device is shown in Figs. 6 and 7. In Fig. 6, it can be seen that the gas flow is generally vertically upwards from the inlet 109 to the outlet 221. In Fig. 7, the flow of the inlet air through the wick duct section 203 is shown, from inlets 202 (arrow A) and through apertures 213, 214 and 215 of the wick heater plate 210 (arrows B, C and D respectively), around the wicks 206.

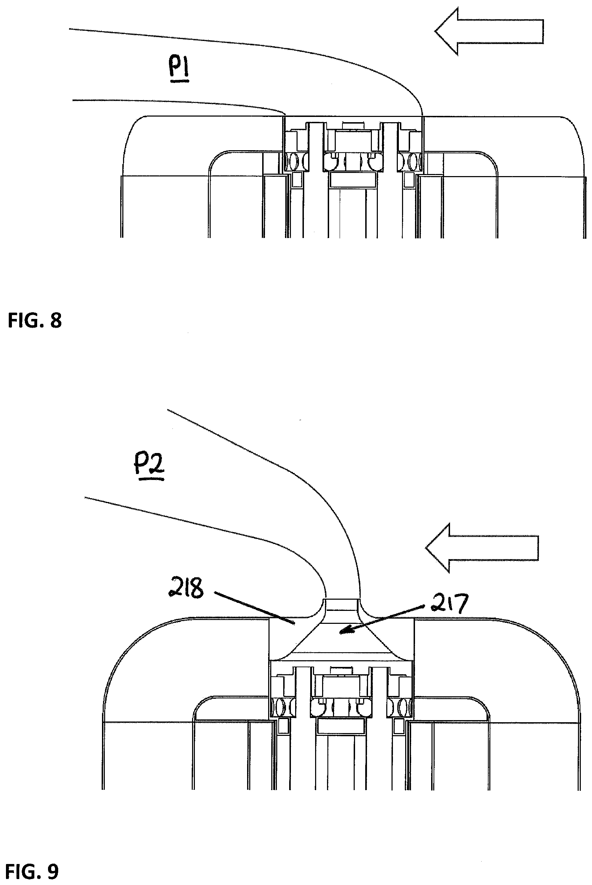

Figs. 8 and 9 illustrate schematically the effect of the outlet duct section 217 configuration on the shape of the vapour plume as it leaves the device, and particularly how far the plume is accelerated away from the device before it begins to disperse in the environment.

Fig. 8 shows a vapour plume profile P1 without an outlet duct fitted and it can be seen that the slower-moving vapour plume is more easily deflected by ambient air current (shown by the arrow) almost as soon as the plume leaves the outlet. The plume therefore passes close to the device and there is a high risk of the vapourised liquid condensing onto the exterior surface of the device or any other surrounding surfaces.

Fig. 9 shows a vapour plume profile P2 with outlet body 218 fitted, which forms outlet duct 217 in accordance with the invention. Due to the increase in speed with which the plume is ejected from the outlet, it moves further away from the device before being deflected by the ambient air current and dispersed in the environment. There is therefore a lower risk of the vapourised liquid condensing onto the exterior surface of the device and mixing of the vapourised material in the external environment is improved.

## Claims

1. Apparatus for vapourising a liquid for supply to an environment, comprising:
a housing (100) having an inlet (109) configured to receive inlet air and an outlet (221) configured to supply a mixture of the inlet air and vapourised liquid to the environment;
a reservoir (204) configured to contain the liquid to be vapourised and a reservoir heater (115) configured to heat the liquid to be vapourised; and
at least one wick (206), one end of the wick being located in the reservoir to receive liquid in use and transport the liquid to the other, exposed end of the wick where the liquid evaporates in use;
wherein the apparatus further includes a duct path between the inlet (109) and the outlet (221) configured to convey the inlet air past the exposed wick end such that the inlet air mixes with the vapourised liquid;
wherein the duct path includes an inlet duct section (108) between the inlet and the wick, and wherein the apparatus further includes an inlet heater (111) in the inlet duct section configured to heat the inlet air and a fan (110) in the inlet duct section configured to draw in the inlet air; and
wherein the duct path includes an outlet duct section (217) between the wick and the outlet, and wherein the outlet duct section is configured to accelerate the flow of the mixture of inlet air and vapourised liquid away from the wick (206) towards the outlet (221).

2. The apparatus of claim 1, wherein the apparatus further includes an inlet plenum chamber (107) external to or upstream of the inlet (109).

3. The apparatus of claim 1 or 2, wherein the outlet duct section (217) includes a conical chamber (219) which reduces in cross-sectional area in the direction of flow towards the outlet to accelerate the flow of the mixture of inlet air and vapourised liquid away from the wick (206) towards the outlet (221).

4. The apparatus of claim 3, wherein the outlet duct section (217) includes a straight section (220) downstream of the conical chamber (219).

5. The apparatus of any preceding claim, further comprising a wick heater (211) for heating the wick (206) at or adjacent to the exposed end of the wick.

6. The apparatus of claim 5, wherein the wick heater is mounted in an apertured wick heater plate (210).

7. The apparatus of any preceding claim, wherein the duct path has a wick duct section (203) between the inlet duct section (108) and the outlet duct section (217), the exposed end of the wick (206) extending into the wick duct section.

8. The apparatus of claims 6 and 7, wherein the apertures (213, 214, 215) in the wick heater plate (210) form part of the wick duct section (203).

9. The apparatus of any preceding claim, further comprising a wick mounting plate (207) for mounting the wick (206).

10. The apparatus of claims 8 and 9, wherein the wick mounting plate (207) is spaced from the wick heater plate (210) and wherein the space between the plates forms part of the wick duct section (203).

11. The apparatus of any preceding claim, further comprising a removable cartridge assembly (200) which includes the reservoir (204) and the wick (206).

12. The apparatus of any preceding claim, wherein the inlet (109) and outlet (221) are arranged on the same vertical axis with the outlet above the inlet.

## Patentansprüche

1. Vorrichtung zum Verdampfen einer Flüssigkeit zur Versorgung einer Umgebung, umfassend:
ein Gehäuse (100), das einen Einlass (109), der dazu konfiguriert ist, Einlassluft aufzunehmen, und einen Auslass (221), der dazu konfiguriert ist, die Umgebung mit einem Gemisch aus der Einlassluft und verdampfter Flüssigkeit zu versorgen, aufweist;
einen Behälter (204), der dazu konfiguriert ist, die zu verdampfende Flüssigkeit zu enthalten, und eine Behälterheizung (115), die dazu konfiguriert ist, die zu verdampfende Flüssigkeit zu erwärmen; und
mindestens einen Docht (206), wobei sich ein Ende des Dochts in dem Behälter befindet, um Flüssigkeit im Gebrauch aufzunehmen und die Flüssigkeit zu dem anderen, freiliegenden Ende des Dochts zu transportieren, wo die Flüssigkeit im Gebrauch verdampft;
wobei die Vorrichtung ferner einen Kanalpfad zwischen dem Einlass (109) und dem Auslass (221) beinhaltet, der dazu konfiguriert ist, die Einlassluft an dem freiliegenden Dochtende vorbei zu befördern, sodass sich die Einlassluft mit der verdampften Flüssigkeit vermischt;
wobei der Kanalpfad einen Einlasskanalabschnitt (108) zwischen dem Einlass und dem Docht beinhaltet und wobei die Vorrichtung ferner eine Einlassheizung (111) in dem Einlasskanalabschnitt, die dazu konfiguriert ist, die Einlassluft zu erwärmen, und ein Gebläse (110) in dem Einlasskanalabschnitt, das dazu konfiguriert ist, die Einlassluft anzusaugen, beinhaltet; und
wobei der Kanalpfad einen Auslasskanalabschnitt (217) zwischen dem Docht und dem Auslass beinhaltet und wobei der Auslasskanalabschnitt dazu konfiguriert ist, die Strömung des Gemischs aus Einlassluft und verdampfter Flüssigkeit weg von dem Docht (206) in Richtung des Auslasses (221) zu beschleunigen.

2. Vorrichtung nach Anspruch 1, wobei die Vorrichtung ferner eine Einlasssammelkammer (107) außerhalb oder stromaufwärts des Einlasses (109) beinhaltet.

3. Vorrichtung nach Anspruch 1 oder 2, wobei der Auslasskanalabschnitt (217) eine konische Kammer (219) beinhaltet, deren Querschnittsfläche in der Strömungsrichtung in Richtung des Auslasses abnimmt, um die Strömung des Gemischs aus Einlassluft und verdampfter Flüssigkeit weg von dem Docht (206) in Richtung des Auslasses (221) zu beschleunigen.

4. Vorrichtung nach Anspruch 3, wobei der Auslasskanalabschnitt (217) einen geraden Abschnitt (220) stromabwärts der konischen Kammer (219) beinhaltet.

5. Vorrichtung nach einem vorhergehenden Anspruch, ferner umfassend einen Dochterhitzer (211) zum Erwärmen des Dochts (206) an oder benachbart zu dem freiliegenden Ende des Dochts.

6. Vorrichtung nach Anspruch 5, wobei der Dochterhitzer in einer Dochterhitzerplatte (210) mit Öffnungen montiert ist.

7. Vorrichtung nach einem vorhergehenden Anspruch, wobei der Kanalpfad einen Dochtkanalabschnitt (203) zwischen dem Einlasskanalabschnitt (108) und dem Auslasskanalabschnitt (217) aufweist, wobei sich das freiliegende Ende des Dochts (206) in den Dochtkanalabschnitt erstreckt.

8. Vorrichtung nach Anspruch 6 und 7, wobei die Öffnungen (213, 214, 215) in der Dochterhitzerplatte (210) einen Teil des Dochtkanalabschnitts (203) bilden.

9. Vorrichtung nach einem vorhergehenden Anspruch, ferner umfassend eine Dochtmontageplatte (207) zum Montieren des Dochts (206).

10. Vorrichtung nach Anspruch 8 und 9, wobei die Dochtmontageplatte (207) von der Dochterhitzerplatte (210) beabstandet ist und wobei der Raum zwischen den Platten einen Teil des Dochtkanalabschnitts (203) bildet.

11. Vorrichtung nach einem vorhergehenden Anspruch, ferner umfassend eine entfernbare Kartuschenbaugruppe (200), die den Behälter (204) und den Docht (206) beinhaltet.

12. Vorrichtung nach einem vorhergehenden Anspruch, wobei der Einlass (109) und der Auslass (221) auf derselben vertikalen Achse mit dem Auslass über dem Einlass angeordnet sind.

## Revendications

1. Appareil pour vaporiser un liquide à fournir à un environnement, comprenant :
un boîtier (100) comportant une entrée (109) conçue pour recevoir de l'air d'entrée et une sortie (221) conçue pour fournir un mélange de l'air d'entrée et de liquide vaporisé à l'environnement ;
un réservoir (204) conçu pour contenir le liquide à vaporiser et un chauffage de réservoir (115) conçu pour chauffer le liquide à vaporiser ; et
au moins une mèche (206), une extrémité de la mèche étant située dans le réservoir pour recevoir le liquide lors de l'utilisation et transporter le liquide vers l'autre extrémité exposée de la mèche où le liquide s'évapore lors de l'utilisation ;
dans lequel l'appareil comprend en outre un chemin de conduit entre l'entrée (109) et la sortie (221) conçu pour transporter l'air d'entrée au-delà de l'extrémité de mèche exposée de telle sorte que l'air d'entrée se mélange au liquide vaporisé ;
dans lequel le chemin de conduit comprend une section de conduit d'entrée (108) entre l'entrée et la mèche, et dans lequel l'appareil comprend en outre un chauffage d'entrée (111) dans la section de conduit d'entrée conçu pour chauffer l'air d'entrée et un ventilateur (110) dans la section de conduit d'entrée conçu pour aspirer l'air d'entrée ; et
dans lequel le chemin de conduit comprend une section de conduit de sortie (217) entre la mèche et la sortie, et dans lequel la section de conduit de sortie est configurée pour accélérer l'écoulement du mélange d'air d'entrée et de liquide vaporisé en éloignement de la mèche (206) vers la sortie (221).

2. Appareil selon la revendication 1, dans lequel l'appareil comprend en outre une chambre de plénum d'entrée (107) externe à ou en amont de l'entrée (109).

3. Appareil selon la revendication 1 ou 2, dans lequel la section de conduit de sortie (217) comprend une chambre conique (219) qui réduit la section transversale dans la direction d'écoulement vers la sortie pour accélérer l'écoulement du mélange d'air d'entrée et de liquide vaporisé en éloignement de la mèche (206) vers la sortie (221).

4. Appareil selon la revendication 3, dans lequel la section de conduit de sortie (217) comprend une section droite (220) en aval de la chambre conique (219).

5. Appareil selon l'une quelconque des revendications précédentes, comprenant en outre un chauffage de mèche (211) pour chauffer la mèche (206) au niveau ou à proximité de l'extrémité exposée de la mèche.

6. Appareil selon la revendication 5, dans lequel le chauffage de mèche est monté dans une plaque de chauffage de mèche à ouvertures (210).

7. Appareil selon l'une quelconque des revendications précédentes, dans lequel le chemin de conduit comporte une section de conduit de mèche (203) entre la section de conduit d'entrée (108) et la section de conduit de sortie (217), l'extrémité exposée de la mèche (206) s'étendant dans la section de conduit de mèche.

8. Appareil selon les revendications 6 et 7, dans lequel les ouvertures (213, 214, 215) dans la plaque de chauffage de mèche (210) font partie de la section de conduit de mèche (203).

9. Appareil selon l'une quelconque des revendications précédentes, comprenant en outre une plaque de montage de mèche (207) pour monter la mèche (206).

10. Appareil selon les revendications 8 et 9, dans lequel la plaque de montage de mèche (207) est espacée de la plaque de chauffage de mèche (210) et dans lequel l'espace entre les plaques fait partie de la section de conduit de mèche (203).

11. Appareil selon l'une quelconque des revendications précédentes, comprenant en outre un ensemble de cartouche amovible (200) qui comprend le réservoir (204) et la mèche (206).

12. Appareil selon l'une quelconque des revendications précédentes, dans lequel l'entrée (109) et la sortie (221) sont agencées sur le même axe vertical avec la sortie au-dessus de l'entrée.
